# EUROPEAN PATENT APPLICATION

(11) **EP 3 992 303 A1**
(43) Date of publication of application: **04.05.2022**
(21) Application number: 19935242.8
(22) Date of filing: 26.06.2019
(51) Int. Cl.: C12Q 1/6869, C12Q 1/6844

(54) **METHOD FOR PREPARING NESTED MULTIPLEX PCR HIGH-THROUGHPUT SEQUENCING LIBRARY AND KIT**

(71) Applicant: MGI Tech Co., Ltd., Shenzhen, Guangdong 518083 (CN)
(72) Inventor: YANG, Lin, Shenzhen, Guangdong 518083 (CN); ZHANG, Yanyan, Shenzhen, Guangdong 518083 (CN); CHEN, Fang, Shenzhen, Guangdong 518083 (CN); JIANG, Hui, Shenzhen, Guangdong 518083 (CN)
(74) Representative: DREISS Patentanwälte PartG mbB
(86) International application number: PCT/CN2019/093066
(87) International publication number: WO 2020/258084

(57) **Abstract**

A method for preparing a high-throughput sequencing library based on nested multiplex PCR and a kit thereof are provided. The method for preparing a high-throughput sequencing library based on nested multiplex PCR includes: amplifying a targeted region by using a forward primer and a reverse primer, where the forward primer is a forward specific sequence, and the reverse primer includes a reverse specific sequence at a 3'-end and a first universal sequencing sequence at a 5'-end; purifying a product of the first round of PCR amplification; and amplifying the purified product by using a nested primer, a first tag primer, and a second universal primer, where the nested primer is located downstream of the forward primer, and includes a second universal sequencing sequence at a 5'-end and a specific sequence at a 3'-end, a 3'-end sequence of the first tag primer is partially or completely the same as the first universal sequencing sequence of the reverse primer at the 5'-end, the first tag primer further includes a first tag sequence, and a 3'-end sequence of the second universal primer is partially or completely the same as the second universal sequencing sequence at the 5'-end of the nested primer. The method for preparing a high-throughput sequencing library based on nested multiplex PCR can effectively avoid dimers generated in the multiplex PCR amplification process, and greatly increase the specificity of PCR amplification by adopting a nested amplification method.

## Description

### FIELD

The present disclosure relates to the technical field of library preparation, and in particular, to a method for preparing high-throughput sequencing libraries based on nested multiplex PCR and a kit for preparing high-throughput sequencing libraries based on nested multiplex PCR.

### BACKGROUND

The demand for re-sequencing of candidate genome segments is increasing with the development of sequencing technology. People pay more attention to sequences than a few SNPs, and the candidate segments may range from 5kb to 10M. The sequencing with the traditional Sanger's method or the targeted region hybridization capture sequencing is expensive, but this problem could be well solved by the use of targeted region capture sequencing. The targeted region capture technology can be roughly divided into two types, i.e., hybridization-based capture sequencing technology and multiplex PCR-based capture technology. By use of multiplex probes or primers, both of them could capture the genome region of interest at one time, and in combination with high-throughput sequencing technology, multiple samples can be sequenced at the same time to obtain the sequence information of the targeted region. Compared with whole-genome sequencing, the target capture sequencing technology greatly reduces costs while screening samples with a large size. The whole-genome sequencing requires cumbersome experimental procedures and high probe costs, which limit its clinical application. In contrast, the target capture sequencing technology has simple experimental operations and strong flexibility, and it is suitable for screening and diagnosis of Mendelian genetic diseases, GWAS candidate segment re-sequencing, QTL mapping segment re-sequencing, precision medical research and application, and other fields.

The high-throughput SNP detection service combines multiplex PCR and high-throughput sequencing technologies to design specific primers for the sites to be detected, and the multiplex PCR amplification carries out in a single tube, with different samples distinguished by different barcode primers. After the samples are mixed, amplicons are sequenced on a sequencing platform, and the different samples are distinguished by means of the sequencing results using a bioinformatics method to finally obtain SNP information for each site. This method is applicable to the genetic research for different purposes, such as disease genome research, tumor genome research, disease and gene association research, clinical molecular diagnosis, etc. In plant genome research, this method can be used for QTL mapping and molecular breeding, which is very suitable for SNP analysis of samples in a large scale.

The multiplex PCR is simple in terms of experimental operations and has a very low cost for a single detection, but it needs to repeatedly test and optimize multiple pairs of primers in the early stage of the experiment, which is time-consuming and labor-intensive. Especially in highly multiplex PCR, due to the complexity of primer sequences, the primers are prone to form primer dimers. The formation of primer dimers will drastically consume raw materials in the PCR reaction system, which results in a plateau phase very soon; and the formed primer dimers will also be sequenced in the subsequent sequencing, generating invalid data, which affects the efficiency of data utilization. The most serious problem is that those primers that are prone to form the primer dimer will seriously affect the amplification efficiency of the targeted amplification region corresponding to the primers, resulting in a low target sequencing depth, and ultimately affecting the uniformity of the entire amplification system. In addition, the specificity of the primers greatly affects the performance of multiplex amplification.

In the multiple amplification, as the number of primer pairs increases, the formation of primer dimers is inevitable. Many companies use additional enzyme treatments to eliminate excess primer dimers. For example, Ampliseq first performs one-step specific amplification, then digests the primer dimers and the specific primer sequence in the amplicon by using enzyme, and finally prepares a library using the product, the whole process of which is cumbersome. Later, Paragon improved this method and invented the Clean Plex dimer elimination technique, which employs specific enzymes to eliminate only primer dimers in the multiplex PCR process, and then performs universal amplification on the digested products by using universal primers. However, regarding non-specific amplification of primers, no better solution is available.

In summary, the shortcomings of the prior art lie in that: (1) the existing methods cannot reduce the formation of primer dimers, the primer dimers can be only eliminated through enzymatic digestion after the primer dimers have been formed, and the primer dimers produced during the multiplex PCR process will greatly affect the efficiency and uniformity of PCR amplification; (2) the existing methods require an additional digestion step, the operation of which is cumbersome; (3) two conventional primers are employed for amplification, and it is difficult to design specific primers for some regions with poor specificity; and (4) existing methods need to be carried out separately in multiple PCR tubes for continuous region amplification.

### SUMMARY

The present disclosure provides a method for preparing high-throughput sequencing libraries based on nested multiplex PCR and a kit for preparing high-throughput sequencing libraries based on nested multiplex PCR, which effectively avoid the formation of dimers during the multiplex PCR amplification. In addition, by adopting a method of nested amplification, the specificity of primers is greatly improved, the data utilization rate is enhanced, and the mutual amplification of the overlapping amplicon primers is effectively inhibited.

According to a first aspect, an embodiment provides a method for preparing a high-throughput sequencing library based on nested multiplex PCR. The method includes: a first round of PCR amplification: amplifying a targeted region by using a forward primer and a reverse primer, wherein the forward primer is a forward specific sequence binding to the targeted region, and the reverse primer includes a reverse specific sequence at a 3'-end binding to the targeted region and a first universal sequencing sequence at a 5'-end; purification of amplification product: purifying a product of the first round of PCR amplification; and a second round of PCR amplification: amplifying the purified product by using a nested primer, a first tag primer, and a second universal primer, wherein the nested primer is located downstream of the forward primer, and the nested primer includes a second universal sequencing sequence at a 5'-end and a specific sequence at a 3'-end, a 3'-end sequence of the first tag primer is partially or completely the same as the first universal sequencing sequence of the reverse primer at the 5'-end, the first tag primer further includes a first tag sequence, and a 3'-end sequence of the second universal primer is partially or completely the same as the second universal sequencing sequence of the nested primer at the 5'-end.

In a preferred embodiment, the second universal primer is a second tag primer, a 3'-end sequence of the second tag primer is partially or completely the same as the second universal sequencing sequence of the nested primer at the 5'-end, and the second tag primer further includes a second tag sequence.

According to a first aspect, another embodiment provides a method for preparing a high-throughput sequencing library based on nested multiplex PCR. The method includes: a first round of PCR amplification: amplifying a targeted region by using a forward primer, a reverse primer, and a first tag primer, wherein the forward primer is a forward specific sequence binding to the targeted region, the reverse primer includes a reverse specific sequence at a 3'-end binding to the targeted region and a first universal sequencing sequence at a 5'-end, a 3'-end sequence of the first tag primer is partially or completely the same as the first universal sequence of the reverse primer at the 5'-end, and the first tag primer further includes a first tag sequence; purification of amplification product: purifying a product of the first round of PCR amplification; and a second round of PCR amplification: amplifying the purified product by using a nested primer, a first universal primer, and a second universal primer, wherein the nested primer is located downstream of the forward primer, the nested primer includes a second universal sequencing sequence at a 5'-end and a specific sequence at a 3'-end, a 3'-end sequence of the first universal primer is partially or completely the same as a 5'-end sequence of the first tag primer, and a 3'-end sequence of the second universal primer is partially or completely the same as the second universal sequencing sequence of the nested primer at the 5'-end.

In a preferred embodiment, the second universal primer is a second tag primer, a 3'-end sequence of the second tag primer is partially or completely the same as the second universal sequencing sequence of the nested primer at the 5'-end, and the second tag primer further includes a second tag sequence.

In a preferred embodiment, the reverse primer further includes a molecular tag sequence.

In a preferred embodiment, the molecular tag sequence is a random sequence of 8 bp to 24 bp.

In a preferred embodiment, the method performs amplification on a plurality of continuous targeted regions, and each of the plurality of continuous targeted regions has the corresponding forward primer, reverse primer, and nested primer, and adjacent amplicon regions have an amplification overlap.

In a preferred embodiment, the forward primer, the reverse primer, and the nested primer are a primer pool composed of a plurality of primers respectively targeting different targeted regions.

In a preferred embodiment, the forward primer of each targeted region and the forward primer of an adjacent targeted region are in opposite amplification directions; the nested primer of each targeted region and the nested primer of an adjacent targeted region are in opposite amplification directions; and the reverse primer of each targeted region and the reverse primer of an adjacent targeted region are in opposite amplification directions.

In a preferred embodiment, the first round of PCR amplification and/or the second round of PCR amplification are each performed for 2 to 30 cycles.

In a preferred embodiment, the first tag sequence and/or the second tag sequence each have a length of 8 bp to 15 bp.

In a second aspect, an embodiment provides a kit for preparing a high-throughput sequencing library based on nested multiplex PCR. The kit includes: a first round of PCR amplification primers, including a forward primer and a reverse primer, wherein the forward primer is a forward specific sequence binding to a targeted region, the reverse primer includes a reverse specific sequence at a 3'-end binding to the targeted region and a first universal sequencing sequence at a 5'-end, the first round of PCR amplification primers are used to perform the first round of PCR amplification on the targeted region; and a second round of PCR amplification primers, including a nested primer, a first tag primer, and a second universal primer, wherein the nested primer is located downstream of the forward primer, the nested primer includes a second universal sequencing sequence at a 5'-end and a specific sequence at a 3'-end, a 3'-end sequence of the first tag primer is partially or completely the same as the first universal sequencing sequence of the reverse primer at the 5'-end, the first tag sequence further includes a first tag sequence, a 3'-end sequence of the second universal primer is partially or completely the same as the second universal sequencing sequence of the nested primer at the 5'-end, and the second round of PCR amplification primers are used to perform the second round of PCR amplification on a purified product of the first round of PCR amplification.

In a preferred embodiment, the second universal primer is a second tag primer, a 3'-end sequence of the second tag primer is partially or completely the same as the second universal sequencing sequence of the nested primer at the 5'-end, and the second tag primer further includes a second tag sequence.

In a second aspect, another embodiment provides a kit for preparing a high-throughput sequencing library based on nested multiplex PCR. The kit includes: a first round of PCR amplification primers, including a forward primer, a reverse primer, and a first tag primer, wherein the forward primer is a forward specific sequence binding to a targeted region, the reverse primer includes a reverse specific sequence at a 3 '-end binding to the targeted region and a first universal sequencing sequence at a 5'-end, a 3'-end sequence of the first tag primer is partially or completely the same as the first universal sequencing sequence of the reverse primer at the 5'-end, the first tag primer further includes a first tag sequence, and the first round of PCR amplification primers are used to perform the first round of PCR amplification on the targeted region; and a second round of PCR amplification primers, including a nested primer, a first universal primer, and a second universal primer, wherein the nested primer is located downstream of the forward primer, the nested primer includes a second universal sequencing sequence at a 5'-end and a specific sequence at a 3'-end, a 3'-end sequence of the first universal primer is partially or completely the same as a 5'-end sequence of the first tag primer, a 3'-end sequence of the second universal primer is partially or completely the same as the second universal sequencing sequence of the nested primer at the 5'-end, and the second round of PCR amplification primers are used to perform the second round of PCR amplification on a purified product of the first round of PCR amplification.

In a preferred embodiment, the second universal primer is a second tag primer, a 3'-end sequence of the second tag primer is partially or completely the same as the second universal sequencing sequence of the nested primer at the 5'-end, and the second tag primer further includes a second tag sequence.

In a preferred embodiment, the reverse primer further includes a molecular tag sequence.

In a preferred embodiment, the molecular tag sequence is a random sequence of 8 bp to 24 bp.

In a preferred embodiment, each of the forward primer, the reverse primer, and the nested primer are a primer pool composed of a plurality of primers respectively targeting a plurality of continuous targeted region, and adjacent amplicon regions have an amplification overlap.

In a preferred embodiment, the forward primer of each targeted region and the forward primer of an adjacent targeted region are in opposite amplification directions; the nested primer of each targeted region and the nested primer of an adjacent targeted region are in opposite amplification directions; and the reverse primer of each targeted region and the reverse primer of an adjacent targeted region are in opposite amplification directions.

In a preferred embodiment, the first tag sequence and/or the second tag sequence each have a length of 8 bp to 15 bp.

The methods and kits for preparing the high-throughput sequencing library based on nested multiplex PCR according to the present disclosure can effectively reduce the formation of primer dimers during the multiplex PCR amplification, and the amplification with three primers can effectively increase the specificity of the product. In addition, the preferred embodiments can effectively suppress invalid amplification fragments generated during the amplification of continuous regions; and the specific primers labeled with molecular tags can uniquely label the original template, thereby tracing a source of the original template and correcting errors.

### BRIEF DESCRIPTION OF DRAWINGS

FIG. 1 is a schematic flowchart of a two-step PCR amplification process known in the prior art;
FIG. 2 is a schematic flowchart of a nested multiplex PCR amplification process in an embodiment of the present disclosure;
FIG. 3 is a schematic diagram of a nested multiplex PCR amplification of continuous regions in an embodiment of the present disclosure;
FIG. 4 illustrates (a) a schematic diagram of a conventional nested PCR primer design in an embodiment of the present disclosure; (b) a schematic diagram of a nested PCR primer design introduced with molecular tags (b), and (c) a schematic diagram of a nested PCR primer design for continuous regions;
FIG. 5 is a schematic diagram illustrating different manners for introducing sample tag in an embodiment of the present disclosure;
FIG. 6 is a schematic diagram illustrating different manners for introducing sample tag in an embodiment of the present disclosure;
FIG. 7 is a diagram illustrating results of uniformity experiments of different amplicons in an embodiment of the present disclosure; and
FIG. 8 is an electrophoresis diagram of a library obtained by a nested multiplex PCR in an embodiment of the present disclosure.

### DESCRIPTION OF EMBODIMENTS

The present disclosure will be further described in detail below through specific embodiments in conjunction with the accompanying drawings. In the following embodiments, many detailed descriptions are used to facilitate the understanding of the present disclosure. However, those skilled in the art could understand that some of the features can be omitted under different circumstances, or can be replaced by other materials and methods.

In addition, the features, operations, or features described in the description can be combined in any appropriate manner to form various implementations. Also, the order of the steps or actions in the method description can also be exchanged or adjusted in a manner that is apparent to those skilled in the art. Therefore, the various sequences in the description and the drawings are only for the purpose of clearly describing a certain embodiment, but are not intended to be a necessary sequence, unless it is otherwise specified that a certain sequence must be followed.

The serial numbers assigned to the features herein, such as "first", "second", etc., are only used to distinguish the described objects and do not have any sequence or technical meaning.

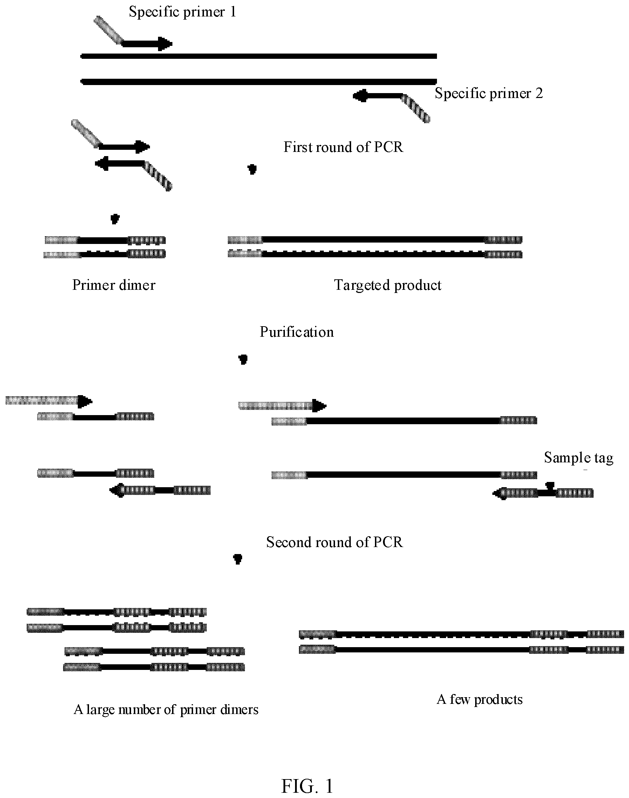

FIG. 1 illustrates a two-step PCR amplification process in the prior art. In the process of specific amplification, two specific primers (i.e., specific primer 1 and specific primer 2 in FIG. 1) carry partial sequences of two sequencing adapters, respectively. These two primers form primer dimers during a first round of PCR amplification. This primer dimers will be amplified in a second round of PCR universal amplification, thereby obtaining a large amount of dimer products, which will affect the sequencing data.

The method of the present disclosure solves the problem that the primer dimer products formed during the specific PCR amplification process are amplified in the subsequent universal PCR amplification process.

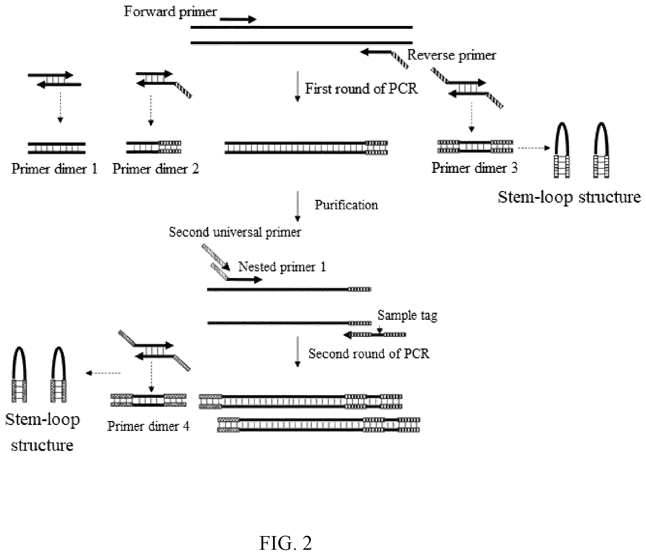

In the method of the present disclosure as illustrated in FIG. 2, during the specific amplification, one specific primer (hereinafter referred to as reverse primer) carries a first universal sequencing sequence, and another specific primer (hereinafter referred to as forward primer) does not carry a sequencing sequence. Two reverse primers form a primer dimer (primer dimer 3 in FIG. 2), and due to the presence of universal sequencing sequences, these primers will form a stem-loop structure during subsequent amplification and will not be amplified. A primer dimer formed by two forward primers (primer dimer 1 in FIG. 2), and a primer dimer formed by the forward primer and the reverse primer (primer dimer 2 in FIG. 2) cannot be used by subsequent universal primers and thus cannot be amplified. In this way, a proportion of primer dimers in the final product of the first round of PCR amplification can be effectively reduced.

As illustrated in FIG. 2, through a semi-nested amplification, the specificity of amplification of the targeted region can be improved. Specifically, in a second round of PCR amplification, a nested primer, which is designed downstream of the forward primer, carries a second universal sequencing sequence. The nested primer and a first tag primer are used to perform the second round of PCR amplification on the obtained product, so as to obtain the final sequencing library. By employing the nested amplification, this method performs a further step of specific selection on the basis of the product of the first round of PCR amplification, thereby increasing the specificity of the final product. This step includes adding the nested primers and the second universal primers for the PCR amplification. During the amplification, only the nested primers may form primer dimers (primer dimer 4 in FIG. 2), and the primer dimers formed by the nested primers will form stem-loop loop structures in the subsequent amplification and will not be amplified, thereby also significantly inhibiting the formation of primer dimers.

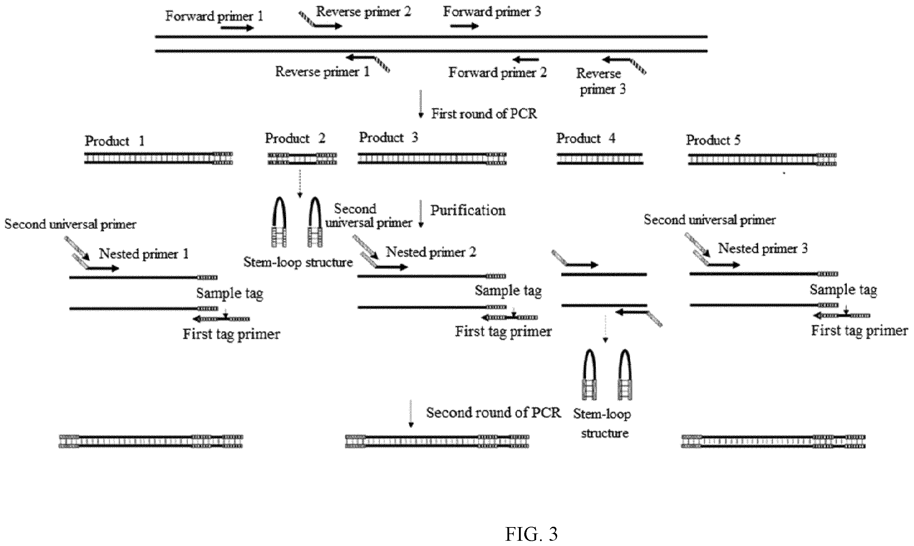

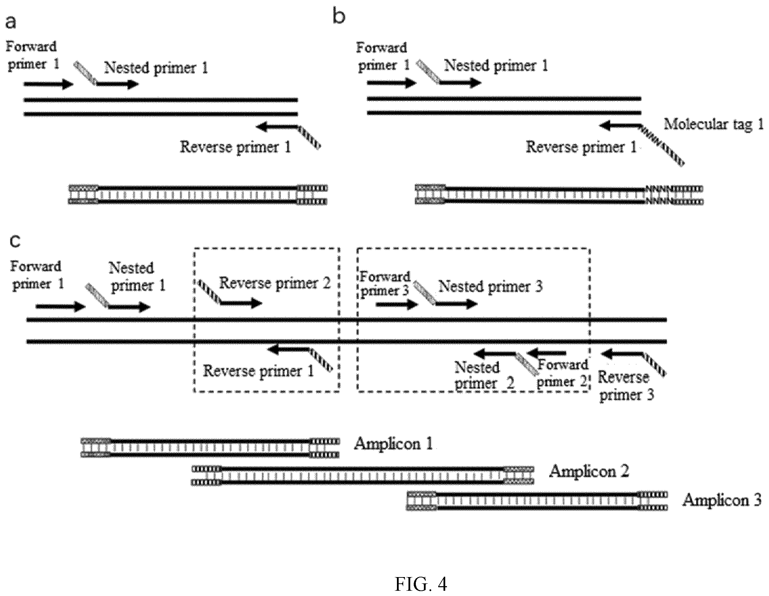

As illustrated in FIG. 3 and FIG. 4, a special primer amplification strategy is used for continuous regions, to avoid mutual amplification of overlapping primers between overlapping amplicons. Specifically, each region includes three primers, i.e., the forward primer, the nested primer, and the reverse primer. It is designed that each forward primer and a next forward primer adjacent thereto can form an amplification overlap, each nested primer and a next nested primers adjacent thereto can form an amplification overlap, and each reverse primer and a next reverse primer can form an amplification overlap (FIG. 4). The forward primers can form a product with each other (product 4 in FIG. 3), which can be used for amplification by the nested primer in the second step of nested amplification. However, because the nested primers each carry the second universal sequencing sequence, the amplification of the product 4 can be inhibited by forming a stem-loop structure during the subsequent amplification. The reverse primers may form a product with each other (product 2 in FIG. 3). Since the reverse primers each carry the first universal sequencing sequence, the amplification of the product 2 can be inhibited by forming a stem-loop structure in the subsequent amplification (FIG. 3). Therefore, none of these overlapping products will not be amplified.

As illustrated in FIG. 4a, for each amplicon, three primers are designed: forward primer, nested primer, and reverse primer, in order to perform the two-step PCR amplification. The nested primer is located downstream of the forward primer, and the nested primer and the forward primer may be overlapped each other or spaced apart from each other. The 5'-end of the nested primer has a universal sequencing sequence segment, the 3'-end of the nested primer is a specific sequence, and the 3'-end sequence is designed to locate upstream of the targeted region to be amplified. The forward primer is designed to locate upstream of the nested primer, the 5'-end of the forward primer can be added with a universal sequencing sequence or not (preferably not). The 5'-end of the reverse primer has a universal sequencing sequence, the 3'-end of the reverse primer is a specific sequence, and the 3'-end sequence is designed to locate downstream of the targeted region to be amplified.

As illustrated in FIG. 4b, if a low-frequency mutation is to be detected, a molecular tag sequence is required to be added between the universal sequence and specific sequence of the reverse primer. The molecular tag can be a random sequence of 8 bp to 24 bp, and thus there may be 4⁸⁻²⁴ kinds of random molecular tags available for labeling the original DNA molecules. When the number of molecular tags is much greater than the number of templates, each template carries a unique molecular tag, through which a source the original DNA template can be traced to remove the amplified duplications and sequencing errors. The introduction of molecular tags to primers will dramatically increase the possibility of dimer formation between the primers, but the primer dimers formed by the present method will be effectively inhibited in the subsequent process.

As illustrated in FIG. 4c, for the amplification of continuous regions, multiple pairs of primers are designed to cover the continuous regions, and adjacent amplicon having an overlapping region, that is, except for the primer sequences in the amplicons, the 3'-end of each amplification is located downstream of the 5'-end of a next amplicon. Each region is amplified with three primers, i.e., the forward primer, the nested primer, and the reverse primer. The forward primer, nested primer, and reverse primer of each targeted region are designed in opposite directions to the forward primer, nested primer, and reverse primer of an adjacent targeted region, respectively, i.e., amplification directions are opposite. For example, a forward primer 1, a nested primer 1, and a reverse primer 1 are arranged sequentially from 5'-end to 3'-end of a first amplicon; then a reverse primer 2, a nested primer 2, and a forward primer 2 are arranged sequentially from 5'-end to 3'-end of a second amplicon; a forward primer 3, a nested primer 3, and a reverse primer 3 are arranged sequentially from 5'-end to 3'-end of a third amplicon, etc., until all amplicons can cover the entire continuous regions. The 3'-end of the reverse primer 2 of the second amplicon is located upstream of the 3'-end of the reverse primer 1, and the 3'-end of the nested primer 3 of the third amplicon is located upstream of the 3'-end of the nested primer 2. With such design, all the sequences in the continuous regions can be amplified into the corresponding amplicons.

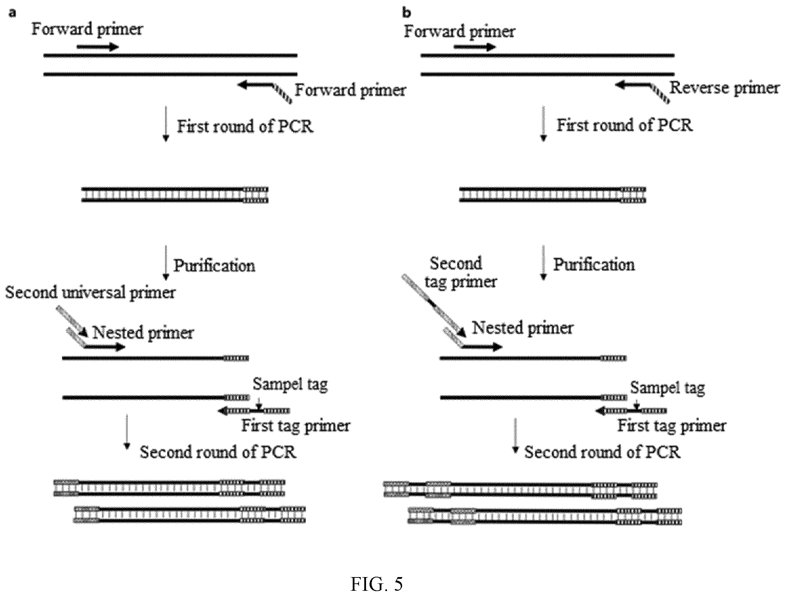

As illustrated in FIG. 5a, in the first round of PCR amplification, 2-30 rounds of PCR amplification are performed using the forward primers and the reverse primers. The obtained product is purified, excess primers and other ions are removed, and then the second round of PCR amplification is performed. In the second round of PCR amplification, 2 to 30 cycles of amplification are performed using the nested primers, the first tag primer, and the second universal primers. Wherein the 3'-end sequence of the first tag primer and the 5'end of the reverse primer are either partially or completely the same in sequence. Each tag primer include a tag sequence of 8 bp to 15 bp in the middle, and the tag sequence can have different sequences on different sequencing platforms, in order to distinguish different samples for subsequent mixed sequencing of multiple samples. The 3'-end sequence of the second universal primer and the 5'-end sequence of the nested primer are either partially or completely the same. In the first cycle of amplification, the amplification is performed using the nested primers and the first tag primer. In the subsequent cycles, the nested primers, the first tag primer and the second universal primers together are used to amplify the target library, so as to finally obtain an amplicon library.

As illustrated in FIG. 5b, for dual-sample tag sequence sequencing, in the second round of PCR amplification, 2 to 30 cycles of amplification are performed using the nested primers, the first tag primer, and the second tag primer. The 3'-end sequence of the first tag sequence is partially or completely the same as the 5'-end sequence of the reverse primer. The 3'-end sequence of the second tag primer is partially or completely the same as the 5'-end sequence of the nested primer. Each tag primer includes a tag sequence of 8bp to 15bp in the middle. The tag sequence can have different sequences on different sequencing platforms, in order to distinguish different samples for the subsequent mixed sequencing of multiple samples. In the first cycle of amplification, the amplification is performed with the nested primers and the first tag primer. In the subsequent cycles, the nested primers, the first tag primer, and the second tag primer together are used to amplify the target library, so as to finally obtain an amplicon library of double sample tags.

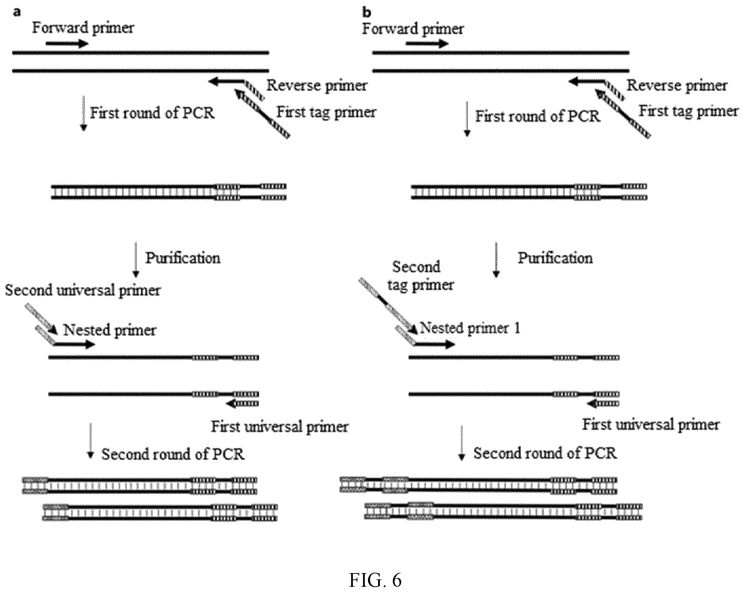

As illustrated in FIG. 6a, for the first round of PCR amplification, in addition to the forward primers and the reverse primers, the first tag primer can also be added. The first tag primer has a tag sequence of 8nt to 15nt in the middle. In this way, the samples can be distinguished in the first round of PCR amplification, and distinguishing the different samples is conducive to the subsequent mixed sequencing of multiple samples. The 3'-end of the first tag primer partially or completely overlaps the 5'-end of the reverse primer. In the first cycle of amplification, the amplification is performed with the forward primers and the reverse primers, and in the subsequent cycles, the amplification is performed with the forward primers, the reverse primers, and the first tag primer. In the second round of PCR amplification, the nested primers, the first universal primers, and the second universal primers are used for 2 to 30 cycles of amplification. The 3'-end of the first universal primer is either partially or completely the same as the 5'-end of the first tag primer. In the first cycle of amplification, the amplification is performed with the first universal primers and the nested primers, and in the subsequent cycles, the amplification is performed with the first universal primers, the second universal primers, and the nested primers together, so as to obtain a sequencing library.

As illustrated in FIG. 6b, for dual-sample tag sequence sequencing, 2 to 30 cycles of amplification is performed with the nested primers, the second tag primer, and the first universal primers in the second round of PCR amplification. The 3'-end of the second tag is partially or completely the same as the 5'-end of the nested primer. The tag primer has a tag sequence of 8 bp to 15 bp in the middle, and the tag sequence has different sequences on different sequencing platforms, in order to distinguish different samples for facilitating the subsequent multi-sample mixed sequencing. The 3'-end sequence of the first universal primer is partially or completely the same as the 5'-end sequence of the first tag primer. In the first cycle of amplification, the amplification is performed with the nested primers and the first universal primers. In the subsequent cycles, the nested primers, the second tag primer, and the first universal primers together are used to amplify the target library to finally obtain an amplicon library of double sample tags.

The technical solutions of the present disclosure are described in detail below by means of specific examples. It should be understood that the examples are only illustrative and should not be construed as limiting the protection scope of the present disclosure.

### Example 1

Primer design: primers of nested multiplex PCR were designed for tumor drugrelated sites. Each amplicon consisted of a forward primer, a nested primer, and a reverse primer. A random sequence of 15nt was introduced into the reverse primer for tracing a source of the template and error correction. These primers were used to prepare a multiplex PCR library of a standard HD701 (Horizon) and complete high-throughput sequencing. The obtained data was analyzed and then the mutation at each targeted site was detected.

1. In a first round of PCR amplification, QIAGEN Multiplex PCR Kit (Cat No./ID: 206143) was used for PCR amplification.

A reagent system listed in Table 1 below was formulated in a 200µL PCR tube:

**[Table 1]**

| Components | Amount |
|---|---|
| Product of previous step (Standard HD701) | 20 µL |
| 2X PCR reaction enzyme | 25 µL |
| Forward primer pool (10 µM) | 2.5 µL |
| Reverse primer pool (10 µM) | 2.5 µL |
| Total | 50 µL |

| | |
|---|---|
| #The forward primer pool and the reverse primer pool are listed in Table 2 and Table 3. | |

**[Table 2] Forward primer pool**

| Primer | Sequence |
|---|---|
| Forward primer 01 | AATGACATAACAGTTATGATTTTGCAG (SEQ ID NO: 1) |
| Forward primer 02 | TGAGTCCTGGCGCTGTGT (SEQ ID NO: 2) |
| Forward primer 03 | TGTTGGATCATATTCGTCCACAA (SEQ ID NO: 3) |
| Forward primer 04 | CCAGCAGGATGAACCGGG (SEQ ID NO: 4) |
| Forward primer 05 | AGTGGAGAAGCTCCCAACC (SEQ ID NO: 5) |
| Forward primer 06 | GACAACCCCCACGTGTGC (SEQ ID NO: 6) |
| Forward primer 07 | GCAGCCAGGAACGTACTG (SEQ ID NO: 7) |
| Forward primer 08 | CCTTACTCATGGTCGGATCACA (SEQ ID NO: 8) |
| Forward primer 09 | AGGGACTAGGCGTGGGAT (SEQ ID NO: 9) |
| Forward primer 10 | GACAAACTCTACGTCTCCTCC (SEQ ID NO: 10) |
| Forward primer 11 | GCCTCAATTCTTACCATCCACAA (SEQ ID NO: 11) |
| Forward primer 12 | GAGACAATGAATTAAGGGAAAATGACAAAG (SEQ ID NO: 12) |
| Forward primer 13 | CATTCGAAAGACYCTAGCCTTAGATA (SEQ ID NO: 13) |

The forward primer pool was formed by mixing 10 µM of the above primers at an equimolar ratio, and Y was a degenerate base.

**[Table 3] Reverse primer pool**

| Primer | Sequence |
|---|---|
| Reverse primer 01 | CGCTTGGCCTCCGACTTNNNNNNNNNNNNNNNNNAGGACTTAGCAAGAAG TTATGGAA (SEQ ID NO: 27) |
| Reverse primer 02 | CGCTTGGCCTCCGACTTNNNNNNNNNNNNNNNNNCATGATGGATGTCACGT TCTCAAA (SEQ ID NO: 27) |
| Reverse primer 03 | CGCTTGGCCTCCGACTTNNNNNNNNNNNNNNNNNNNNGAATATAAACTTGTGGT AGTTGGAGCT (SEQ ID NO: 29) |
| Reverse primer 04 | CGCTTGGCCTCCGACTTNNNNNNNNNNNNNNNNTGGTTACATCCCTCTCT GCT (SEQ ID NO: 30) |
| Reverse primer 05 | CGCTTGGCCTCCGACTTNNNNNNNNNNNNNNNNTTATACACCGTGCCGAA CGC (SEQ ID NO: 31) |
| Reverse primer 06 | CGCTTGGCCTCCGACTTNNNNNNNNNNNNNNNNTGTTCCCGGACATAGTC CAG (SEQ ID NO: 32) |
| Reverse primer 07 | CGCTTGGCCTCCGACTTNNNNNNNNNNNNNNNNNNGCCTCCTTCTGCATGGT ATTCTTT (SEQ ID NO: 33) |
| Reverse primer 08 | CGCTTGGCCTCCGACTTNNNNNNNNNNNNNNNNGAGAATGGGTACTCAC GTTTCCTT (SEQ ID NO: 34) |
| Reverse primer 09 | CGCTTGGCCTCCGACTTNNNNNNNNNNNNNNNNNNTGTGGAAAAGTCCCAAT GGAACTATC (SEQ ID NO: 35) |
| Reverse primer 10 | CGCTTGGCCTCCGACTTNNNNNNNNNNNNNNNNNNNNCTCTCTCCACCAGAGCG A (SEQ ID NO: 36) |
| Reverse primer 11 | CGCTTGGCCTCCGACTTNNNNNNNNNNNNNNNNAATAGGTGATTTGGTC TAGCTACA (SEQ ID NO: 37) |
| Reverse primer 12 | CGCTTGGCCTCCGACTTNNNNNNNNNNNNNNNNATTTAGCACTTACCTG TGACTCCA (SEQ ID NO: 38) |
| Reverse primer 13 | CGCTTGGCCTCCGACTTNNNNNNNNNNNNNNNTGTGTGGAAGATCCAAT CCATT (SEQ ID NO: 39) |

The reverse primer pool was formed by mixing 10 µM of the above primers at an equimolar ratio.

The first round of PCR amplification was performed according to the procedure as listed in Table 4 below:

**[Table 4]**

| Temperature | Duration | The number of cycles |
|---|---|---|
| 95°C | 2min | 1 cycle |
| 95°C | 10s | |
| 62°C | 2min | 2 cycles |
| 72°C | 30s | |
| 72°C | 5min | 1 cycle |

80 µL of XP magnetic beads were added to the obtained PCR product for purification (Agencourt AMPure XP magnetic beads from Beckman, Cat No: A63881), and the obtained product was dissolved in 20 µL of TE solution.

2. For the second round of PCR amplification, QIAGEN Multiplex PCR Kit (Cat No./ID: 206143) was used for PCR amplification.

A reagent system as listed in Table 5 was prepared in a 200µL PCR tube:

**[Table 5]**

| Components | Amount |
|---|---|
| Product of previous step | 20 µL |
| 2X PCR reaction enzyme | 25 µL |
| Nested primer pool | 2.5 µL |
| Second universal primer | 2.5 µL |
| First tag primer | 2.5 µL |
| Total | 50 µL |

| | |
|---|---|
| #The nested primer pool is illustrated in Table 6. | |

**[Table 6] Nested primer pool**

| Primer | Sequence |
|---|---|
| Nested primer 01 | ACATGGCTACGATCCGACTTTCAGTGTTACTTACCTGTCTTGTC (SEQ ID NO: 14) |
| Nested primer 02 | ACATGGCTACGATCCGACTTTCAGGATGGTGGATGTGGG (SEQ ID NO: 15) |
| Nested primer 03 | ACATGGCTACGATCCGACTTGCTGTATCGTCAAGGCACTC (SEQ ID NO: 16) |
| Nested primer 04 | ACATGGCTACGATCCGACTTACCCCAATGCAGCGAACAA (SEQ ID NO: 17) |
| Nested primer 05 | ACATGGCTACGATCCGACTTAGCTCTCTTGAGGATCTTGAAGG (SEQ ID NO: 18) |
| Nested primer 06 | ACATGGCTACGATCCGACTTCTGCCTCACCTCCACCGT (SEQ ID NO: 19) |
| Nested primer 07 | ACATGGCTACGATCCGACTTAACACCGCAGCATGTCAA (SEQ ID NO: 20) |
| Nested primer 08 | ACATGGCTACGATCCGACTTTGTGATTTTGGTCTAGCCAGAG (SEQ ID |
| | NO: 21) |
| Nested primer 09 | ACATGGCTACGATCCGACTTGATGATGGGCTCCCGGAA (SEQ ID NO: 22) |
| Nested primer 10 | ACATGGCTACGATCCGACTTCGTCTCCTCCGACCACTGT (SEQ ID NO: 23) |
| Nested primer 11 | ACATGGCTACGATCCGACTTGATCCAGACAACTGTTCAAACTG (SEQ ID NO: 24) |
| Nested primer 12 | ACATGGCTACGATCCGACTTGCAATTTCTACACGAGATCCTCT (SEQ ID NO: 25) |
| Nested primer 13 | ACATGGCTACGATCCGACTTGCAAGAGGCTTTGGAGTATTTCATG (SEQ ID NO: 26) |

The nested primer pool was formed by mixing 10µM of the above primers at an equimolar ratio.

First tag primer: TGTGAGCCAAGGAGTTNNNNNNNNNN^{#}TTGTCTTCCTAAGACCGCTTGGCCTCCG ACTT (SEQ ID NO: 40), # where the N is the tag sequence, which is random base.

Second universal primer: /5Phos/#GAACGACATGGCTACGATCCGACTT (SEQ ID NO: 41), where the 5'-end of the primer is phosphorylated and used for the circularization of the MGI platform.

The first round of PCR amplification was performed according to the procedure in Table 7 below.

**[Table 7]**

| Temperature | Duration | The number of cycles |
|---|---|---|
| 95°C | 2min | 1 cycle |
| 95°C | 10s | 24 cycles |
| 62°C | 2min | |
| 72°C | 30s | |
| 72°C | 5min | 1 cycle |

80 µL of XP magnetic beads were added to the obtained PCR product for purification (Agencourt AMPure XP magnetic beads from Beckman, Cat No. A63881), and the obtained product was dissolved in 20 µL of TE solution.

### 3. Library quality inspection

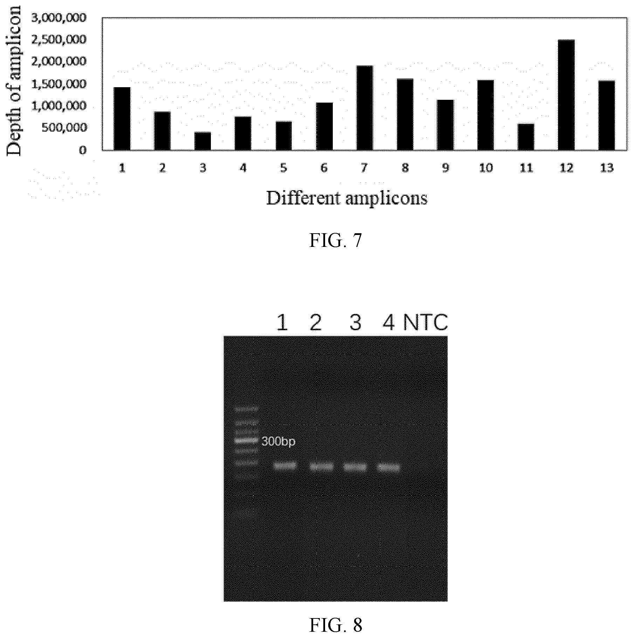

The library was tested, the band range was between 150 bp and 200 bp, and the results are listed in FIG. 8.

### 4. Sequencing

All the obtained products were standardized and mixed in equal amounts, and the mixed library was subjected to parallel sequencing, with a sequencing platform MGISEQ-2000 and a sequencing type PE100.

### 5. Data analysis

The analysis steps include basic steps such as filtering of the adapter primer sequences, alignment and so on. The basic information obtained is listed in Table 8. Then, GATK was used to detect mutations at the targeted site (Table 9).

**[Table 8] Statistics of sequencing data**

| No. | Raw data | Mapping rate | Capture rate | Average depth | Uniformity 0.2X |
|---|---|---|---|---|---|
| Sample 1 | 8349037 | 99.85% | 99.72% | 1277933 | 100% |
| Sample 2 | 11265885 | 99.85% | 99.68% | 1711470 | 100% |
| Sample 3 | 10184135 | 99.88% | 99.73% | 1542768 | 100% |
| Sample 4 | 15748586 | 99.89% | 99.75% | 2389774 | 100% |

**[Table 9] Detection of mutation sites**

| Mutation sites | Theoretical detection value | Actual detection value 1 | Actual detection value 2 | Actual detection value 3 | Actual detection value 4 |
|---|---|---|---|---|---|
| E545K | 9.0% | 9.7% | 9.3% | 9.6% | 8.6% |
| H1047R | 17.5% | 18.7% | 18.4% | 17.2% | 15.8% |
| D816V | 10.0% | 9.2% | 11.0% | 9.2% | 10.2% |
| G719S | 24.5% | 26.7% | 23.5% | 24.5% | 26.5% |
| T790M | 1.0% | 0.9% | 1.0% | 1.0% | 1.0% |
| L858R | 3.0% | 3.1% | 2.9% | 3.3% | 3.1% |
| V600E | 10.5% | 9.6% | 11.2% | 9.8% | 10.3% |
| G13D | 15.0% | 14.7% | 14.7% | 16.5% | 13.8% |
| G12D | 6.0% | 6.5% | 6.3% | 6.4% | 6.2% |

It can be seen from FIG. 8 that, the products obtained by using the nested multiplex PCR do not contain the primer dimers and the non-specific products. Table 8 indicates that both the mapping rate and the capture rate in the sequencing data can reach 99%, the uniformity of 0.2X average depth can reach 100%, and the depth of each amplicon is within 7 times (FIG. 7), indicating an excellent amplification performance. The mutation detection performed on the targeted sites indicates that the mutation frequencies actually detected in all samples are close to the detected mutation frequency of the standard, and the actual values are approximately equal to 1 ± 0.1 times the theoretical value (Table 9).

The above applies specific examples to explain the present disclosure, which are only used to help understanding the present disclosure, but not mean to limit the present disclosure. Those skilled in the art can also make several simple deductions, modifications, or substitutions based on the concept of the present disclosure.

## Claims

1. A method for preparing a high-throughput sequencing library based on nested multiplex PCR, the method comprising:
a first round of PCR amplification: amplifying a targeted region by using a forward primer and a reverse primer, wherein the forward primer is a forward specific sequence binding to the targeted region, and the reverse primer comprises a reverse specific sequence binding to the targeted region at a 3'-end and a first universal sequencing sequence at a 5'-end;
purification of amplification product: purifying a product of the first round of PCR amplification; and
a second round of PCR amplification: amplifying the purified product by using a nested primer, a first tag primer, and a second universal primer, wherein the nested primer is located downstream of the forward primer, the nested primer comprises a second universal sequencing sequence at a 5'-end and a specific sequence at a 3'-end, a 3'-end sequence of the first tag primer is partially or completely the same as the first universal sequencing sequence of the reverse primer at the 5'-end, the first tag primer further comprises a first tag sequence, and a 3'-end sequence of the second universal primer is partially or completely the same as the second universal sequencing sequence of the nested primer at the 5'-end.

2. The method according to claim 1, wherein the second universal primer is a second tag primer, a 3'-end sequence of the second tag primer is partially or completely the same as the second universal sequencing sequence of the nested primer at the 5'-end, and the second tag primer comprises a second tag sequence.

3. A method for preparing a high-throughput sequencing library based on nested multiplex PCR, the method comprising:
a first round of PCR amplification: amplifying a targeted region by using a forward primer, a reverse primer, and a first tag primer, wherein the forward primer is a forward specific sequence binding to the targeted region, the reverse primer comprises a reverse specific sequence binding to the targeted region at a 3'-end and a first universal sequencing sequence at a 5'-end, a 3'-end sequence of the first tag primer is partially or completely the same as the first universal sequence of the reverse primer at the 5'-end, and the first tag primer further comprises a first tag sequence;
purification of amplification product: purifying a product of the first round of PCR amplification; and
a second round of PCR amplification: amplifying the purified product by using a nested primer, a first universal primer, and a second universal primer, wherein the nested primer is located downstream of the forward primer, the nested primer comprises a second universal sequencing sequence at a 5'-end and a specific sequence at a 3'-end, a 3'-end sequence of the first universal primer is partially or completely the same as a 5'-end sequence of the first tag primer, and a 3'-end sequence of the second universal primer is partially or completely the same as the second universal sequencing sequence of the nested primer at the 5'-end.

4. The method according to claim 3, wherein the second universal primer is a second tag primer, a 3'-end sequence of the second tag primer is partially or completely the same as the second universal sequencing sequence of the nested primer at the 5'-end, and the second tag primer comprises a second tag sequence.

5. The method according to any one of claims 1 to 4, wherein the reverse primer further comprises a molecular tag sequence.

6. The method according to claim 5, wherein the molecular tag sequence is a random sequence of 8 bp to 24 bp.

7. The method according to any one of claims 1 to 4, wherein a plurality of continuous targeted regions is amplified, and each of the plurality of continuous targeted regions has the corresponding forward primer, reverse primer, and nested primer, and adjacent amplicon regions have an amplification overlap.

8. The method according to claim 7, wherein the forward primer, the reverse primer, and the nested primer are a primer pool composed of a plurality of primers respectively targeting different targeted regions of the plurality of continuous targeted regions.

9. The method according to claim 7, wherein the forward primer of each targeted region and the forward primer of an adjacent targeted region are in opposite amplification directions; the nested primer of each targeted region and the nested primer of an adjacent targeted region are in opposite amplification directions; and the reverse primer of each targeted region and the reverse primer of an adjacent targeted region are in opposite amplification directions.

10. The method according to any one of claims 1 to 4, wherein the first round of PCR amplification and/or the second round of PCR amplification are each performed for 2 to 30 cycles.

11. The method according to any one of claims 1 to 4, wherein the first tag sequence and/or the second tag sequence each have a length of 8 bp to 15 bp.

12. A kit for preparing a high-throughput sequencing library based on nested multiplex PCR, the kit comprising:
a first round of PCR amplification primers, comprising a forward primer and a reverse primer, wherein the forward primer is a forward specific sequence binding to a targeted region, the reverse primer comprises a reverse specific sequence binding to the targeted region at a 3'-end and a first universal sequencing sequence at a 5'-end, the first round of PCR amplification primers are used to perform a first round of PCR amplification on the targeted region; and
a second round of PCR amplification primers, comprising a nested primer, a first tag primer, and a second universal primer, wherein the nested primer is located downstream of the forward primer, the nested primer comprises a second universal sequencing sequence at a 5'-end and a specific sequence at a 3'-end, a 3'-end sequence of the first tag primer is partially or completely the same as the first universal sequencing sequence of the reverse primer at the 5'-end, the first tag sequence further comprises a first tag sequence, a 3'-end sequence of the second universal primer is partially or completely the same as the second universal sequencing sequence of the nested primer at the 5'-end, and the second round of PCR amplification primers are used to perform a second round of PCR amplification on a purified product of the first round of PCR amplification.

13. The kit according to claim 12, wherein the second universal primer is a second tag primer, a 3'-end sequence of the second tag primer is partially or completely the same as the second universal sequencing sequence of the nested primer at the 5'-end, and the second tag primer comprises a second tag sequence.

14. A kit for preparing a high-throughput sequencing library based on nested multiplex PCR, the kit comprising:
a first round of PCR amplification primers, comprising a forward primer, a reverse primer, and a first tag primer, wherein the forward primer is a forward specific sequence binding to a targeted region, the reverse primer comprises a reverse specific sequence binding to the targeted region at a 3'-end and a first universal sequencing sequence at a 5'-end, a 3'-end sequence of the first tag primer is partially or completely the same as the first universal sequencing sequence of the reverse primer at the 5'-end, the first tag primer further comprises a first tag sequence, and the first round of PCR amplification primers are used to perform a first round of PCR amplification on the targeted region; and
a second round of PCR amplification primers, comprising a nested primer, a first universal primer, and a second universal primer, wherein the nested primer is located downstream of the forward primer, the nested primer comprises a second universal sequencing sequence at a 5'-end and a specific sequence at a 3'-end, a 3'-end sequence of the first universal primer is partially or completely the same as a 5'-end sequence of the first tag primer, a 3'-end sequence of the second universal primer is partially or completely the same as the second universal sequencing sequence of the nested primer at the 5'-end, and the second round of PCR amplification primers are used to perform a second round of PCR amplification on a purified product of the first round of PCR amplification.

15. The kit according to claim 14, wherein the second universal primer is a second tag primer, a 3'-end sequence of the second tag primer is partially or completely the same as the second universal sequencing sequence of the nested primer at the 5'-end, and the second tag primer comprises a second tag sequence.

16. The kit according to any one of claims 12 to 15, wherein the reverse primer further comprises a molecular tag sequence.

17. The kit according to claim 16, wherein the molecular tag sequence is a random sequence of 8 bp to 24 bp.

18. The kit according to any one of claims 12 to 15, wherein the forward primer, the reverse primer, and the nested primer are a primer pool composed of a plurality of primers respectively targeting a plurality of continuous targeted region, and adjacent amplicon regions have an amplification overlap.

19. The kit according to claim 18, wherein the forward primer of each targeted region and the forward primer of an adjacent targeted region are in opposite amplification directions; the nested primer of each targeted region and the nested primer of an adjacent targeted region are in opposite amplification directions; and the reverse primer of each targeted region and the reverse primer of an adjacent targeted region are in opposite amplification directions.

20. The kit according to any one of claims 12 to 15, wherein the first tag sequence and/or the second tag sequence each have a length of 8 bp to 15 bp.


SEQUENCE LISTING

<110>  MGI TECH CO., LTD.

<120>  METHOD FOR PREPARING  HIGH-THROUGHPUT SEQUENCING LIBRARY BASED ON
NESTED MUTIPLEX PCR AND KIT THEREOF

<130>  19P28353

<160>  41

<170>  PatentIn version 3.3

<210>  1
<211>  27
<212>  DNA
<213>  Artificial sequence

<400>  1
aatgacataa cagttatgat tttgcag                                          27


<210>  2
<211>  18
<212>  DNA
<213>  Artificial sequence

<400>  2
tgagtcctgg cgctgtgt                                                    18


<210>  3
<211>  23
<212>  DNA
<213>  Artificial sequence

<400>  3
tgttggatca tattcgtcca caa                                              23


<210>  4
<211>  18
<212>  DNA
<213>  Artificial sequence

<400>  4
ccagcaggat gaaccggg                                                    18


<210>  5
<211>  19
<212>  DNA
<213>  Artificial sequence

<400>  5
agtggagaag ctcccaacc                                                   19


<210>  6
<211>  18
<212>  DNA
<213>  Artificial sequence

<400>  6
gacaaccccc acgtgtgc                                                    18


<210>  7
<211>  18
<212>  DNA
<213>  Artificial sequence

<400>  7
gcagccagga acgtactg                                                    18


<210>  8
<211>  22
<212>  DNA
<213>  Artificial sequence

<400>  8
ccttactcat ggtcggatca ca                                               22


<210>  9
<211>  18
<212>  DNA
<213>  Artificial sequence

<400>  9
agggactagg cgtgggat                                                    18


<210>  10
<211>  21
<212>  DNA
<213>  Artificial sequence

<400>  10
gacaaactct acgtctcctc c                                                21


<210>  11
<211>  23
<212>  DNA
<213>  Artificial sequence

<400>  11
gcctcaattc ttaccatcca caa                                              23


<210>  12
<211>  30
<212>  DNA
<213>  Artificial sequence

<400>  12
gagacaatga attaagggaa aatgacaaag                                       30


<210>  13
<211>  26
<212>  DNA
<213>  Artificial sequence

<400>  13
cattcgaaag acyctagcct tagata                                          26


<210>  14
<211>  56
<212>  DNA
<213>  Artificial sequence


<220>
<221>  misc_feature
<222>  (18)..(32)
<223>  n is a, c, g, or t

<400>  14
cgcttggcct ccgacttnnn nnnnnnnnnn nnaggactta gcaagaagtt atggaa          56


<210>  15
<211>  56
<212>  DNA
<213>  Artificial sequence


<220>
<221>  misc_feature
<222>  (18)..(32)
<223>  n is a, c, g, or t

<400>  15
cgcttggcct ccgacttnnn nnnnnnnnnn nncatgatgg atgtcacgtt ctcaaa          56


<210>  16
<211>  59
<212>  DNA
<213>  Artificial sequence


<220>
<221>  misc_feature
<222>  (18)..(32)
<223>  n is a, c, g, or t

<400>  16
cgcttggcct ccgacttnnn nnnnnnnnnn nngaatataa acttgtggta gttggagct       59


<210>  17
<211>  52
<212>  DNA
<213>  Artificial sequence


<220>
<221>  misc_feature
<222>  (18)..(32)
<223>  n is a, c, g, or t

<400>  17
cgcttggcct ccgacttnnn nnnnnnnnnn nntggttaca tccctctctg ct              52

<210>  18
<211>  52
<212>  DNA
<213>  Artificial sequence

<220>
<221>  misc_feature
<222>  (18)..(32)
<223>  n is a, c, g, or t

<400>  18
cgcttggcct ccgacttnnn nnnnnnnnnn nnttatacac cgtgccgaac gc          52

<210>  19
<211>  52
<212>  DNA
<213>  Artificial sequence

<220>
<221>  misc_feature
<222>  (18)..(32)
<223>  n is a, c, g, or t

<400>  19
cgcttggcct ccgacttnnn nnnnnnnnnn nntgttcccg gacatagtcc ag          52

<210>  20
<211>  56
<212>  DNA
<213>  Artificial sequence

<220>
<221>  misc_feature
<222>  (18)..(32)
<223>  n is a, c, g, or t

<400>  20
cgcttggcct ccgacttnnn nnnnnnnnnn nngcctcctt ctgcatggta ttcttt      56

<210>  21
<211>  56
<212>  DNA
<213>  Artificial sequence

<220>
<221>  misc_feature
<222>  (18)..(32)
<223>  n is a, c, g, or t

<400>  21
cgcttggcct ccgacttnnn nnnnnnnnnn nngagaatgg gtactcacgt ttcctt      56

<210>  22
<211>  58

<212>  DNA
<213>  Artificial sequence

<220>
<221>  misc_feature
<222>  (18)..(32)
<223>  n is a, c, g, or t

<400>  22
cgcttggcct ccgacttnnn nnnnnnnnnn nntgtggaaa agtcccaatg gaactatc        58

<210>  23
<211>  50
<212>  DNA
<213>  Artificial sequence

<220>
<221>  misc_feature
<222>  (18)..(32)
<223>  n is a, c, g, or t

<400>  23
cgcttggcct ccgacttnnn nnnnnnnnnn nnctctctcc accagagcga                50

<210>  24
<211>  57
<212>  DNA
<213>  Artificial sequence

<220>
<221>  misc_feature
<222>  (18)..(32)
<223>  n is a, c, g, or t

<400>  24
cgcttggcct ccgacttnnn nnnnnnnnnn nnaataggtg attttggtct agctaca         57

<210>  25
<211>  57
<212>  DNA
<213>  Artificial sequence

<220>
<221>  misc_feature
<222>  (18)..(32)
<223>  n is a, c, g, or t

<400>  25
cgcttggcct ccgacttnnn nnnnnnnnnn nnattttagc acttacctgt gactcca         57

<210>  26
<211>  54
<212>  DNA
<213>  Artificial sequence

<220>
<221>  misc_feature
<222>  (18)..(32)
<223>  n is a, c, g, or t

<400>  26
cgcttggcct ccgacttnnn nnnnnnnnnn nntgtgtgga agatccaatc catt          54


<210>  27
<211>  44
<212>  DNA
<213>  Artificial sequence

<400>  27
acatggctac gatccgactt tcagtgttac ttacctgtct tgtc                    44


<210>  28
<211>  39
<212>  DNA
<213>  Artificial sequence

<400>  28
acatggctac gatccgactt tcaggatggt ggatgtggg                          39


<210>  29
<211>  40
<212>  DNA
<213>  Artificial sequence

<400>  29
acatggctac gatccgactt gctgtatcgt caaggcactc                         40


<210>  30
<211>  39
<212>  DNA
<213>  Artificial sequence

<400>  30
acatggctac gatccgactt accccaatgc agcgaacaa                          39


<210>  31
<211>  43
<212>  DNA
<213>  Artificial sequence

<400>  31
acatggctac gatccgactt agctctcttg aggatcttga agg                     43


<210>  32
<211>  38
<212>  DNA
<213>  Artificial sequence

<400>  32
acatggctac gatccgactt ctgcctcacc tccaccgt                           38

<210>  33
<211>  38
<212>  DNA
<213>  Artificial sequence

<400>  33
acatggctac gatccgactt aacaccgcag catgtcaa                              38


<210>  34
<211>  42
<212>  DNA
<213>  Artificial sequence

<400>  34
acatggctac gatccgactt tgtgattttg gtctagccag ag                         42


<210>  35
<211>  38
<212>  DNA
<213>  Artificial sequence

<400>  35
acatggctac gatccgactt gatgatgggc tcccggaa                              38


<210>  36
<211>  39
<212>  DNA
<213>  Artificial sequence

<400>  36
acatggctac gatccgactt cgtctcctcc gaccactgt                             39


<210>  37
<211>  43
<212>  DNA
<213>  Artificial sequence

<400>  37
acatggctac gatccgactt gatccagaca actgttcaaa ctg                        43


<210>  38
<211>  43
<212>  DNA
<213>  Artificial sequence

<400>  38
acatggctac gatccgactt gcaatttcta cacgagatcc tct                        43


<210>  39
<211>  45
<212>  DNA
<213>  Artificial sequence

<400>  39
acatggctac gatccgactt gcaagaggct ttggagtatt tcatg                      45

<210>  40
<211>  58
<212>  DNA
<213>  Artificial sequence

<220>
<221>  misc_feature
<222>  (17)..(26)
<223>  n is a, c, g, or t

<400>  40
tgtgagccaa ggagttnnnn nnnnnnttgt cttcctaaga ccgcttggcc tccgactt        58

<210>  41
<211>  25
<212>  DNA
<213>  Artificial sequence

<400>  41
gaacgacatg gctacgatcc gactt                                            25